# EUROPEAN PATENT APPLICATION

(11) **EP 2 280 022 A1**
(43) Date of publication of application: **02.02.2011**
(21) Application number: 09166967.1
(22) Date of filing: 31.07.2009
(51) Int. Cl.: C07K 5/08, C07K 7/02, A61P 25/00, A61P 25/16, A61P 25/28, A61P 35/00

(54) **Cripto blocking molecules and therapeutic uses thereof**

(71) Applicant: CONSIGLIO NAZIONALE DELLE RICERCHE, 00185 Roma (IT)
(72) Inventor: Minchiotti, Gabriella, 80131, Napoli (IT); Ruvo, Menotti, 83058, Avellino (IT); De Falco, Sandro, 80136, Napoli (IT); Marasco, Daniela, 80134 Napoli (IT); Lonardo, Enza, 00185, Roma (IT); Parish, Clare, Parkville 3010 (AU); Arenas, Ernest, 17177 Stockholm (SE)
(74) Representative: Capasso, Olga

(57) **Abstract**

The present invention concerns a monomeric or multimeric tripeptide molecule able of inhibiting Cripto signalling in embryonic stem cells, thereby enhancing dopamine specification and differentiation, after transplantation in animal models of Parkinson's disease, as well as reducing tumor formation, and uses thereof.

## Description

### Technical background

Recent breakthroughs in stem cell research have identified embryonic stem cells (ESC) as unique tools for drug/small molecule development ^{1, 2-3, 4} and cell replacement therapy (CRT) in neurodegenerative disorders, including Parkinson's disease (PD) ⁵⁻⁸. PD has been identified as a priority target for drug development and CRT because the classical signs of the disease (tremor, rigidity, and hypokinesia) are caused by the progressive degeneration of a circumscribed cell population, substantia nigra dopaminergic (DA) neurons. The idea of using stem cells for CRT in PD patients has been fuelled by the finding that human ventral midbrain fetal tissue grafts, under specific conditions, improve the symptoms and reduce the need of L-DOPA in this patients ⁹⁻¹¹. Pluripotent stem cells have become an attractive cell source for CRT because of their capacity to generate large numbers of DA neurons and induce functional recovery in Parkinsonian mice ¹²⁻¹⁴. However, several challenges need to be overcome in order to allow ESCs as tools for CRT. Amongst them, it is important to underscore the need to: (i) achieve homogenous populations of correctly specified, functional midbrain DA neurons, and (ii) improve the safety of ESC transplantation by eliminating tumor formation. Efforts to elucidate the molecular programs that direct DA specification and differentiation have lead to the identification of several factors including engrailed-1 ¹⁵, Shh and FGF8 ¹⁶, Nurr1 ¹⁷, Lmx1b ¹⁸, Pitx3 ¹⁹, Ngn2 ²⁰, Lmx1a ²¹, Otx2 ²², Wnt1 ^{15, 23}, Foxa2 ²⁴; and Wnt5a ^{25, 26}. While several of these factors have been implemented in ESC cultures ^{13,14,27,28}, the identification of molecules capable of promoting or allowing controlled DA specification and differentiation of ESCs in the absence of tumors, remain to be identified.

A major challenge in the field of stem cell transplantation is to reduce the risk of tumor formation by undifferentiated ESCs or progenitors after transplantation^{27, 45, 46}. This challenge has lead to the development of cell sorting strategies to eliminate unwanted cells and/or select the desired cell types ⁴⁷⁻⁴⁹. However, the quest to identify a strategy to provide additional safety measures and intrinsically prevent tumor formation, eliminate proliferating and/or non-neuronal cells, as well as increase neuronal differentiation without genetic modification of ESCs, still remains. In this study the authors report an innovative strategy to improve ESC CRT by: (i) restricting the fate of ESCs and preventing mesoderm formation, (ii) limiting the tumorigenic potential and, (iii) improving the engraftment and DA differentiation of ESC-derived neural cells and their functional outcome in Parkinsonian rats. This strategy was based on the identification (by a combinatorial screening) of a novel peptide that antagonizes Cripto signalling, which favours the acquisition of cardiomyocytes versus neural fate of ESCs ^{29, 35}. Although, combinatorial screenings have been successfully used in the last two decades to identify small molecules/peptides for use in drug discovery and cell biology ¹, the value of such approaches in the stem cell field is just emerging. Recently, cell-based screenings identified synthetic molecules that regulate stem cell differentiation/proliferation, which have been used as probes to study the underlying biology of stem cells ^{1, 2, 50}. Whilst many basic questions regarding identification of targets and mechanisms of action still remain to be answered, it has become evident that such synthetic molecules can lead to significant advances in stem cell biology and therapy. In particular, peptides are known to be optimal probes to target the extracellular domains of membrane receptors because of their typical high affinity and selectivity, minimal drug-drug interactions, low accumulation capacity and low toxicity ⁴⁶.

Authors previously found that deletion of *cripto* in ESCs increased their DA differentiation *in vivo,* in the absence of tumors ²⁹. Cripto is a GPI-anchored receptor that belongs to the EGF-CFC protein family and acts as modulator of TGF-b family signalling ^{30-32, 36, 37}.

Cripto binds the TGF-b ligand Nodal, or related Gdf1 or -3, and the Activin type I receptor ALK-4, thereby activating a complex formed with Activin type IIB serine/threonine kinase (ActRIIB) receptor ³⁰⁻³². Upon receptor activation, the intracellular effectors Smad2/Smad3 become phosphorylated and together with Smad4 translocate into the nucleus to regulate gene expression ^{33, 34}. Consistent with that, Cripto acts through the Nodal/ALK-4 (activin type IB receptor) pathway to negatively regulate neural differentiation and permit the entry of ESCs into cardiac lineage ³⁵. In support of these findings, disruption of *cripto* in ESCs enhanced neurogenesis and DA differentiation *in vitro,* and when grafted at low density, *cripto-l-*ESCs suppress tumor formation and promote functional recovery in parkinsonian rats ²⁹. Whilst ESC deficient in selected genes such as *cripto* are valuable tools in a research setting and provide pivotal information about development and cell therapies, the use of genetically modified cell lines in a clinical setting remains a less favourable option.

WO 2004/083375 describes a method to induce stem cell differentiation into neuronal cells by exposing the cells to an inhibitor of the Cripto protein. However this application does not describe any peptide able to block the interaction between the EGF-CFC Cripto protein and the ALK-4 receptor for controlling ESC differentiation and decreasing tumor formation.

Then by blocking the interaction between the EGF-CFC Cripto protein and the ALK-4 receptor using small molecules/peptides represent an ideal target for controlling ESC differentiation and decreasing tumor formation ^{29, 51}. In the present study, the authors screened a combinatorial peptide library made of non-natural amino acids, to identify competitors of Cripto/ALK-4 interaction, in an ELISA -based assay. Following library deconvolution by an iterative process, Cripto BP was identified and found to selectively bind Cripto and prevent its *in vitro* binding to ALK-4 receptor. Moreover, Cripto BP prevented Cripto-dependent Smad2 phosphorylation, blocked cardiomyogenesis and favoured neurogenesis in ESCs, mimicking the phenotype of *cripto-*/*-* ESCs ^{29, 35}. Thus, our *in vitro* results support the idea that, despite the function of ALK-4 as a receptor for multiple TGFβ-related ligands ⁵², the neutralizing activities that the authors detect are the result of specific targeting of the Cripto protein. Moreover, since peptides are extremely flexible molecules, have a high recognition surface, and are highly resistant to proteases, it has been suggested that they can be applied in functional assays and in a broad range of applications both *in vitro* and *in vivo.*

In agreement with this, the authors hereby show that the administration of Cripto BP improved the effects of a combination of developmental factors, including Shh, FGF8, bFGF and Wnt5a, and increased the number of ESC-derived DA neurons both *in vitro* and *in vivo,* compared to CP. Indeed, pre-treatment of ESC with Cripto BP prior to grafting consistently and efficiently increased the generation of midbrain-like DA neurons *in vivo* and reduced tumor formation. Moreover, these findings could be further enhanced by additional infusion of Cripto BP into the brain parenchyma. Cripto BP treatment resulted in an enhanced behavioural recovery in the amphetamine-induced rotation test. Thus, our results collectively indicate that Cripto BP reliably improves both the functional outcome and the safety of ESC grafting in Parkinsonian rats. In the future, further chemical modifications of Cripto BP and improvements in the differentiation protocols for human ESCs may lead to the successful translation of human ESCs in a clinical setting and to improvements in the treatment of PD.

All together the present invention provides the first *in vivo* evidence that a small peptide, capable of antagonizing Cripto signalling in an ESC screening assay, can contribute to fulfil functional (increased neural induction and DA differentiation) and safety (reduction of tumor formation) requirements for ESCs to be a viable option for CRT.

Authors therefore endeavoured to develop novel compounds capable of inhibiting Cripto signalling in ESCs, thereby enhancing DA specification and differentiation, as well as reducing tumor formation after transplantation in animal models of neurodegenerative disease.

### Description of the invention

The authors screened a combinatorial tripeptide library built with non-natural amino acids on a multimeric scaffold, which ensured enhanced recognition capabilities deriving from the large recognition surface and the high flexibility of the final compounds ^{38, 39}.

Peptide mixtures were utilized as competitors of Cripto/ALK-4 interaction in an ELISA-based competition assay. Selected peptides were evaluated for their ability to inhibit Cripto-induced Smad2 phosphorylation in ESCs and promote neuronal differentiation. These assays allowed to identify a tetrameric tripeptide, named Cripto Blocking Peptide (Cripto BP). The action/activity of this peptide was further tested on DA differentiation of ESCs *in vitro.* Moreover, Cripto BP treated ESCs were transplanted in Parkinsonian rats, to examine their ability to promote safe and functional recovery, thereby improving ESC-based strategies for CRT.

Then embryonic stem cells (ESCs) can be combined with a high throughput screen to identify a Cripto blocking peptide (Cripto BP or BP) that improves ESC CRT in a rat model of Parkinson's disease (PD). Cripto BP was identified by virtue of its ability to prevent Cripto/ALK-4 receptor interaction and interfere with Cripto-dependent Smad2 phosphorylation in ESCs. Moreover, Cripto BP prevented cardiomyogenesis and favoured neuroectoderm formation by ESCs, promoting the specification and differentiation of midbrain dopaminergic neurons. Cripto BP -treated ESCs, when transplanted into the striatum of PD rats, reduced tumor prevalence and size and enhanced DA neurons integration and functional recovery. Screening and ESC-based assays can be used to identify novel molecules such as Cripto BP which improve both safety and efficiency of ESC in CRT models for PD.

It is therefore an object of the present invention a tripeptide molecule of formula (I):

Y₁-(X1)-(X2)- (X3) (I)

wherein
Y₁ is an amino-terminal group or an acetylated amino-terminal group;
X1 belongs to the group of (R)- or (S)-Glu, (R)- or (S)-Met, (R)- or (S)-Met[O], (R)- or (S)-Met[O]₂, where Glu is glutamic acid, Met is methionine, Met[O] is methionine sulphoxide, Met[O]₂ is methionine sulphone, (R)- or (S)- refer to the R or S absolute configuration;
X2 and X3 are an (R)- or (S)-(S-aryl)-Cysteine, where S-aryl means any aryl group directly linked to the thiol group of the cysteine; (R)- or (S)- indicates the absolute configuration on the chiral center of the cysteine; and are equal or different;
or a combination thereof.

Examples of aryl groups are as follows: phenyl, benzyl, 2-phenyl-ethyl, 1-phenyl-ethyl, 2-methyl-phenyl, 3-methyl-phenyl, 4-methyl-phenyl, 2,3-dimethylphenyl, 2,4-dimethylphenyl, 2,5-dimethylphenyl, 2,6-dimethylphenyl, 2-benzyl-ethyl, 1-benzyl-ethyl, 2-methyl-benzyl, 3-methyl-benzyl, 4-methyl-benzyl, 2,3-dimethyl-benzyl, 2,4-dimethyl-benzyl, 2,5-dimethyl-benzyl, 2,6-dimethyl-benzyl, naphtyl and alkyl-substituted derivatives, di-phenyls and alkyl-substituted di-phenyls.

In a preferred aspect X1 is S-Met[O], R-Glu or S-Met[O]₂, X2 and X3 are S-Cys(Bzl) (S-benzylcysteine).

In a preferred embodiment the tripeptide molecule of the invention is comprised in a multimeric branched tripeptide of formula I wherein each tripeptide is attached to a branching scaffold, preferably comprising from 2 to 8 of said tripeptides.

In a preferred embodiment the branching scaffold has the formula (II):

Z-Y₂ (II)

wherein
Z consists of one or more trifunctional amino acids (TFAA), being equal or different each other;
Y₂ is a hydroxyl- or amino group, an amino acid, or a peptide linked via an amide bond to the terminal carbonyl group of Z;
Y₂ is equal or different from the tripeptide of formula (I).

Preferably the TFAA comprises as functional groups two amino groups and one carboxyl group.

Preferably each tripeptide of formula (I) is linked to an amino group of said TFAA. More preferably said trifunctional amino acid (TFAA) has the formula (III): 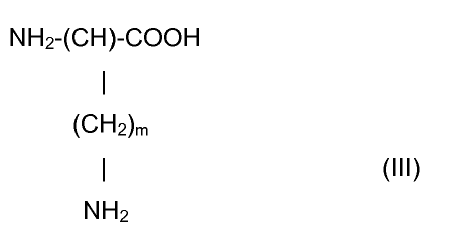 wherein
m is an integer from 1 to 4; the amino acid is a R- or S-enantiomer.

In a preferred embodiment Z consists of 3 or 7 TFAAs, forming a (TFAA)₂-TFAA- or (TFAA)₄-(TFAA)₂-TFAA-branching scaffold, respectively.

In a preferred embodiment said TFAAs are R- or S-enantiomers of 2,3-diaminopropionic acid, of 2,4-diaminobutyric acid, of ornithine, of 2,5-diaminopentanoic acid, of lysine, or of 2,6-diaminoesanoic acid.

In a preferred embodiment Y₂ is a small or a non-bulky amino acid, such as glycine or alanine; or tripeptide formed of any combination of R-arginine, S-arginine, R-lysine, S-lysine.

In a most preferred embodiment the multimeric tripeptide molecule has the formula 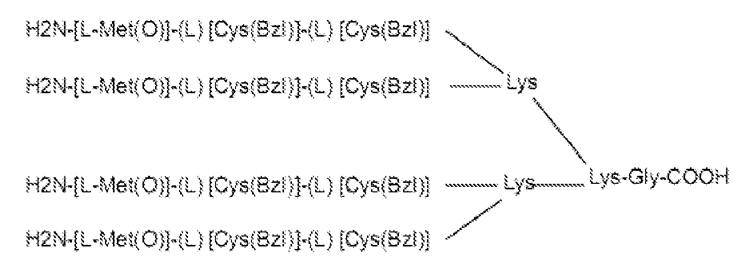 wherein [L-Met(O)] is (S)-Methionine-sulphoxide and [Cys(Bzl)] is (S)-S-benzylated-Cysteine.

It is an object of the invention the use of the molecule as above disclosed for specifically and selectively binding to Cripto protein and acting as a competitor for the binding of Cripto protein to the Alk-4 receptor and as inhibitor of Cripto-dependent Smad2 phosphorylation.

It is an object of the invention the use of the molecule as above disclosed for selectively inhibiting the Cripto-dependent differentiation process of stem cells and induce their differentiation to neuronal cells, and said neuronal induced stem cells as above treated.

It is an object of the invention the molecule or the neuronal induced stem cells for medical use, preferably for the treatment of a neurodegenerative disease, more preferably for the treatment of Parkinson disease; alternatively for the treatment of Cripto-dependent tumor formation.

It is an object of the invention a pharmaceutical composition comprising the molecule as above disclosed or the neuronal induced stem cells as above disclosed, and proper excipients, adjuvants and/or diluents, also associated with other therapeutic compounds acting on Cripto, or to ameliorate side effects induced by other therapeutic compounds wherein said other therapeutic compounds may or may not act on Cripto.

The present invention refers to stem cells. They can be of mammalian origin, preferably of mouse or human origin, as i.e. human embryonic stem cells and/or pluripotent human stem cells (iPS), not derived from destruction human embryos, i.e. by :
i) Therapeutic Cloning/ Somatic Cell Nuclear Transfer, i.e. insertion of somatic cell nucleus into a donor oocyte;
ii) Nuclear Reprogramming of adult cells to iPS (induced Pluripotent Stem Cells).

Examples of methods for obtaining such cells are disclosed in :
- Yamanaka S. Strategies and new developments in the generation of patient-specific pluripotent stem cells. Cell Stem Cell. 2007 Jun 7;1(1):39-49. Two "reprogramming" strategies for the generation of pluripotent stem cells from somatic cells have been studied extensively: nuclear transfer to oocytes and fusion with ES cells. Patient-specific pluripotent cells could potentially also be generated by the spontaneous reprogramming of bone marrow cells, spermatogonial cells. A third overall type of strategy arose from the demonstration that pluripotent stem (iPS) cells can be generated from mouse fibroblasts by the introduction of four transcription factors (Oct-3/4, Sox2, c-Myc, and KLF4). Recent work has underlined the potential of this strategy by improving the efficiency of the process and demonstrating that iPS cells can contribute to many different tissues in vivo, including the germline. Taken together, these studies underscore the crucial roles of transcription factors and chromatin remodeling in nuclear reprogramming.
- Takahashi K, Yamanaka S. Induction of pluripotent stem cells from mouse embryonic and adult fibroblast cultures by defined factors. Cell. 2006 Aug 25;126(4):652-5. This manuscript report the first evidence of iPS generation from adult somatic cells.
- Takahashi K, Okita K, Nakagawa M, Yamanaka S. Induction of pluripotent stem cells from fibroblast cultures. Nat Protoc. 2007;2(12):3081-9.
- Takahashi K, Tanabe K, Ohnuki M, Narita M, Ichisaka T, Tomoda K, Yamanaka S. Induction of pluripotent stem cells from adult human fibroblasts by defined factors. Cell. 2007 Nov 30;131 (5):861-72.
- Masui S, Nakatake Y, Toyooka Y, Shimosato D, Yagi R, Takahashi K, Okochi H, Okuda A, Matoba R, Sharov AA, Ko MS, Niwa H. Pluripotency governed by Sox2 via regulation of Oct3/4 expression in mouse embryonic stem cells. Nat Cell Biol. 2007 Jun;9(6):625-35.
- Saito S, Sawai K, Murayama Y, Fukuda K, Yokoyama K. Nuclear transfer to study the nuclear reprogramming of human stem cells. Methods Mol Biol. 2008;438:151-69.
- Aoi T, Yae K, Nakagawa M, Ichisaka T, Okita K, Takahashi K, Chiba T, Yamanaka S. Generation of Pluripotent Stem Cells from Adult Mouse Liver and Stomach Cells. Science. 2008 Feb 14 [E pub ahead of print].
- Nakagawa M, Koyanagi M, Tanabe K, Takahashi K, Ichisaka T, Aoi T, Okita K, Mochiduki Y, Takizawa N, Yamanaka S. Generation of induced pluripotent stem cells without Myc from mouse and human fibroblasts. Nat Biotechnol. 2008 Jan;26(1):101-6. E pub 2007 Nov 30.

### Detailed description of the invention

The present invention will be now described by means of non limiting examples as illustrated in the following figures. In this document D can be used interchangeably with (R) and L can be used interchangeably with (S).

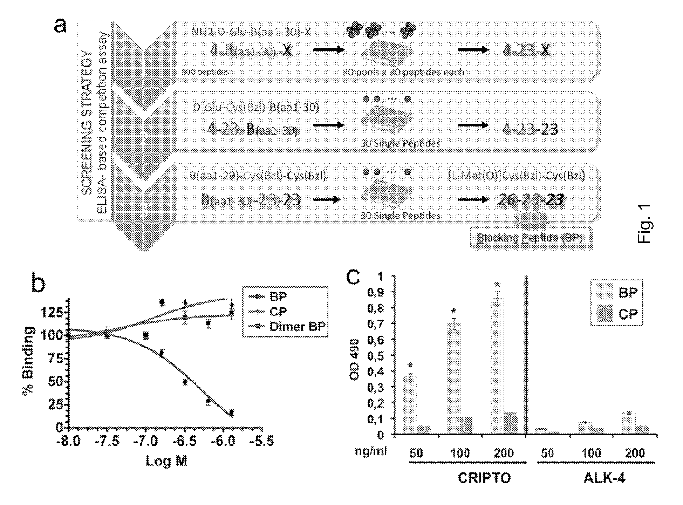

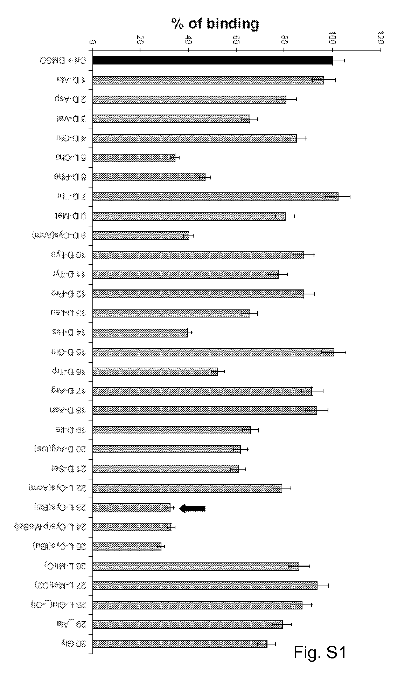

**Figure 1 (a)** Schematic representation of the iterative screening strategy. (1) NH2-D-Glu (D-Glutamic acid, also denoted as "4", see **Table S1)** is the common N-terminal residue, B identifies the fixed residue, which denotes one out of 30 aminoacids (aa1-30, **Table S1)** and X indicates random positions (4-B₍₁₋₃₀₎-X). The total complexity of the first library was therefore of 30²= 900 different peptides. In the first screening round (1), 4.23.X identifies the first active pool, where "23" indicates L-Cysteine(S-benzyl) [L-Cys(Bzl)]. In the second round of screening (2) the pool 4-23-X was re-synthesized in 30 single peptides (4-23-B₍₁₋₃₀₎), and the residue "23" was identified in the third position (4-23-23). Finally, thirty single peptides (B₍₁₋₃₀₎-23-23) were submitted to the third screening round (3), which identified the residue "26", where "26" indicates L-Methionine-sulphoxide [L-Met(O)]. The 26-23-23 peptide is also referred to as Cripto Blocking Peptide (BP) **(b)** Dose dependent inhibition of Cripto/ALK-4 interaction exerted by Cripto BP, Control Peptide (CP; i.e. tetrameric tripeptide D-Ser-Ala-Cha) and Dimeric Peptide (DP; i.e. dimeric tripeptide 26-23-23) in ELISA-based competition assays (n=3). **(c)** Dose dependent interaction of Cripto or ALK-4 to Cripto BP. The indicated peptides (BP and CP) were coated (50µM) on the plates and increasing amounts of either Cripto or ALK-4 receptor were used in binding, ranging from 50 to 200ng/ml. Cripto but not ALK-4 bound Cripto BP; whereas, both Cripto and ALK-4 failed to bind the control peptide (CP). Mean ± SD, *P< 0.0005.

**Figure S1.** Screening of the (D-Glu)-B-X library. In the first round of screening, to reduce the library complexity, the first residue of the library was fixed, i.e. the aminoacid D-Glutamic acid (D-Glu). The library was made from 30 pools, each of them composed by 30 peptides that differ for the second aminoacidic position. Pools were tested in the ELISA-based competition assay with a molar excess of 30000-fold (calculated for each single peptide) over Cripto (8.6 x10⁻¹⁰ M).

**Figure S2.** Dose-dependent inhibition of selected pools. Pools identified in the first screening were tested in the ELISA-based competition assay with a molar excess of 5000, 12500 and 30000-fold **(a)** and 200, 1000 and 5000-fold **(b).** Molar excess was calculated for each single peptide over Cripto (8.6 x10⁻¹⁰ M).

**Figure S3.** Screening of the (D-Glu)-[L-Cys(Bzl)]-B library. Thirty single peptides were synthesised and tested in the ELISA-based competition assay with a molar excess of 1000-fold over Cripto (8.6 x10⁻¹⁰ M).

**Figure S4. (a)** Screening of the B-[L-Cys(Bzl)]-[L-Cys(Bzl)] library. Thirty single peptides were synthesised and tested in the ELISA-based competition assay with a molar excess of 1000-fold over Cripto (8.6 x10⁻¹⁰ M). **(b)** Dose-dependent inhibition of selected peptides. Peptides were tested in the ELISA-based competition assay with a molar excess of 500, 1000, 2000, 3000 and 4000 -fold. Molar excess was calculated for each single peptide over Cripto (8.6 x10⁻¹⁰ M).

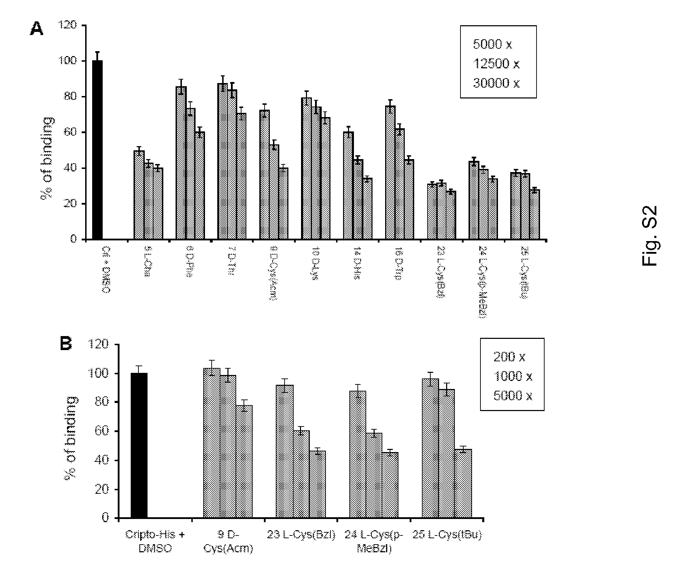

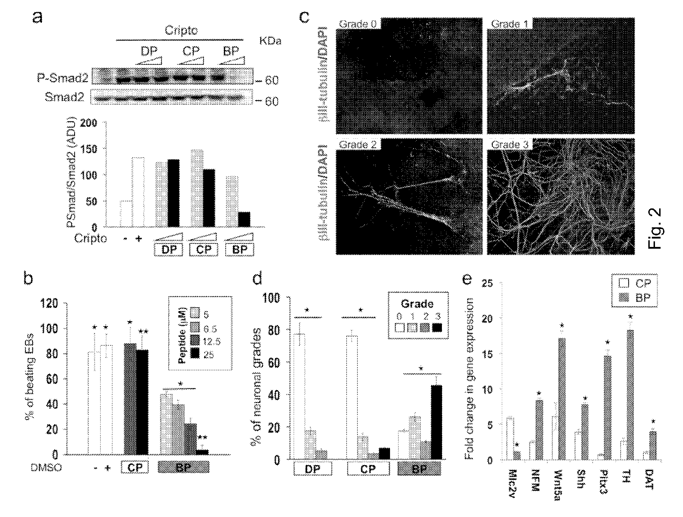

**Figure 2****.** Cripto BP prevents Smad2 phosphorylation and redirects the fate of ESCs. **(a)** Two day old *cripto^{-l-}* EBs were stimulated with recombinant Cripto protein (0.5µg/ml) in the presence of either 2.5µM or 25µM of the peptides: DP (dimeric 26-23-23), CP (control tetrameric D-Ser-Ala-Cha) and BP (tetrameric 26-23-23). Cripto BP, but not CP or DP inhibits Cripto -induced Smad2 phosphorylation, as shown by Western blot, using anti phospho-Smad2 antibodies. Levels of total Smad2 were also compared. Smad2 phosphorylation was expressed as ratio between arbitrary densitometric units (ADU) of P-Smad2 and Smad2. The data show a representative experiment, n=3. **(b)** Cripto BP (but not CP, DP or DMSO) prevented cardiomyogenesis in differentiated EBs. Morphological analysis was performed on day 13 (n=∼60EBs/group; data are mean ± SD; *P<0.001, versus DMSO and CP; **P<0.0001, versus CP). **(c)** Grades of neuronal differentiation of wild type EBs, as arbitrarily defined. Grade 0: absence of neurons; Grade1: few, isolated neurons immunoreactive to β-III tubulin antibodies; Grade 2: small areas of β-III tubulin-ir cells; Grade 3: dense network of β-III tubulin-ir cells. Nuclei were visualized by DAPI counterstaining. Magnification 20x. **(d)** BP (but not CP or DP) promoted full neuronal differentiation in wild-type EBs (degree of neuronal differentiation scored according to panel **c).** Two -days old wild-type EBs were treated with 25µM of the indicated peptides and immunofluorescence analysis was performed on day 13 [n=∼60EBs/group, data are mean ± SD; *P<0.05 (Grade 0 compared to grade 2 or 3)]. **(e).** Expression analysis of cardiac and neuronal markers. Two-day old EBs were treated either with the indicated peptides at 25µM (CP; BP) or vehicle (DMSO) and the expression profile was analysed on day 13, by QRT-PCR. The neuronal marker (NF-M) and markers involved in Dopaminergic (DA) maturation (Wnt5a, Shh, Pitx3, TH, DAT) were up regulated; whereas the cardiac marker (MIc2v) was down regulated. Data are mean ± SEM, n≥3; fold change in gene expression was relative to (DMSO) control; *P is versus CP-treated group.

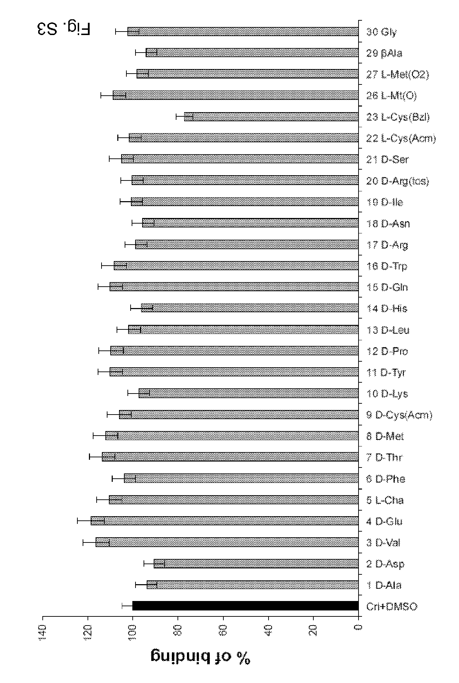

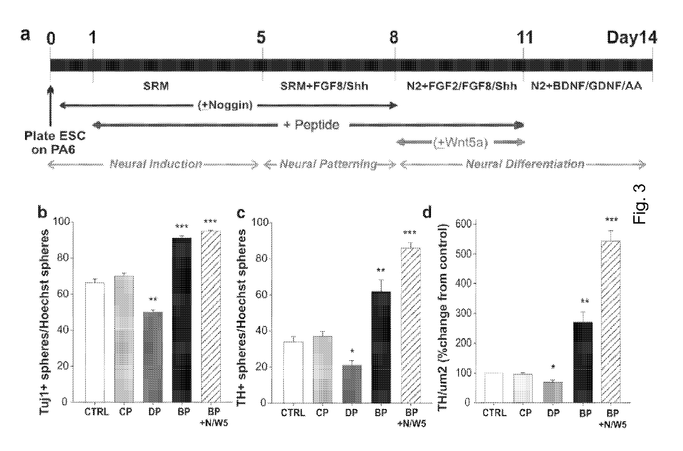

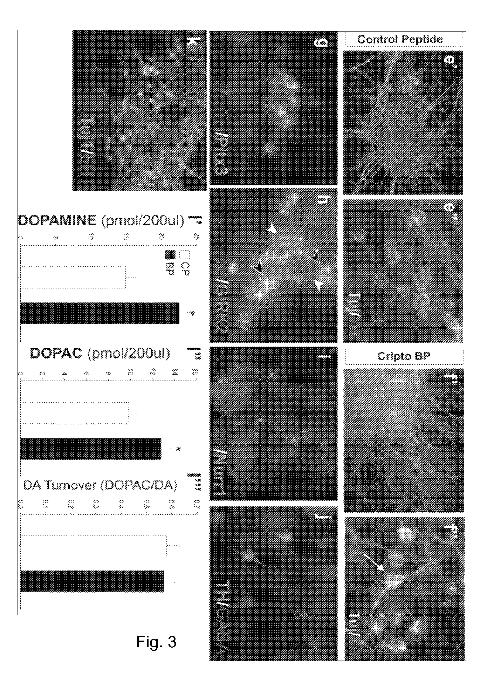

**Figure 3****.** Cripto BP enhances neuronal induction and dopaminergic differentiation. **(a)** *In vitro* differentiation scheme. ESCs were seeded at low density onto a stromal feeder layer for 14 days in the presence of sequential morphogens and mitogens (Shh, FGF2, FGF8, Noggin; N, Wnt5a; W5) as well as the presence or absence of the Control Peptide (CP) or Cripto blocking peptides (BP). **(b)** *In vitro* differentiation of ESCs in the presence of BP significantly increased the percentage of colonies containing neurons (TUJ1). **(c)** Cripto BP significantly increased the proportion of colonies containing dopaminergic neurons (TH), and **(d)** the number of DA neurons/colony. Mean ± SD, *p<0.05, **p<0.01, ***p<0.0001 versus CTRL. Photomicrographs illustrating TH+ neurons within neuronal enriched ESC colonies following **(e)** CP administration, and **(f)** Cripto BP treatment. **(g)** TH/Pitx3, and **(h)** TH/GIRK2 and **(i)** TH/Nurr1 co-localized staining within the cultures confirmed the midbrain dopaminergic phenotype of the TH neurons. The differentiation protocol was selective for DA neurons with few cells of other neuronal subpopulations present, and these cells did not colocalize with TH **(j)** TH/GABA and **(k)** 5HT/Tuj1. Cripto BP treated ESC cultures showed significantly increased dopamine **(l)** and DOPAC **(l")** release *in vitro* following KCI evoked release. Data represents mean ± SEM, n=3, * p<0.05).

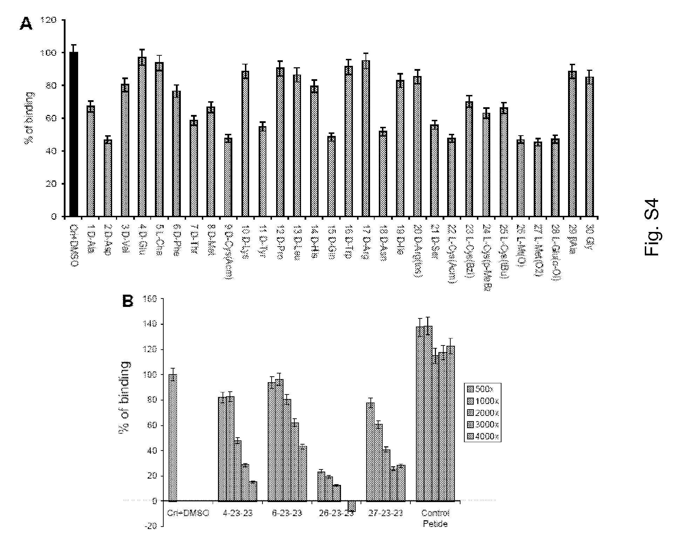

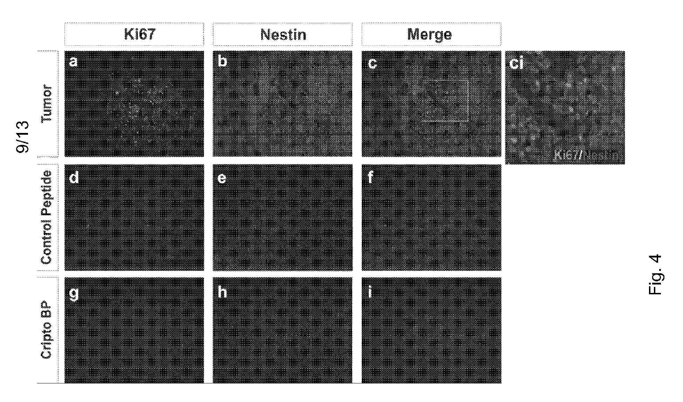

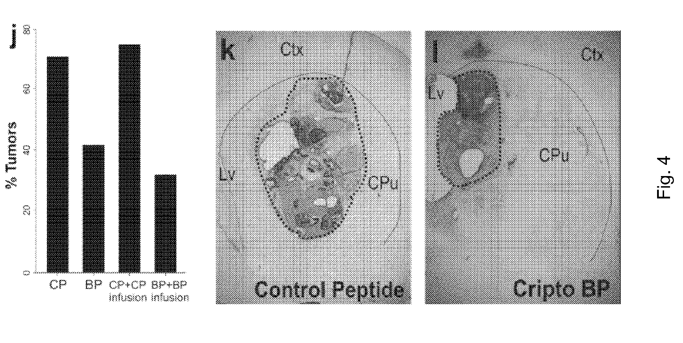

**Figure 4****.** Cripto BP reduces the incidence and size of tumors following mESC transplantation. **(a)** Tumors or potential tumor-like cells could be identified by the presence of proliferating cells (Ki67) and **(b)** undifferentiated cells (Nestin) within the graft. **(c)** Merged image. **(ci)** High power magnification of proliferative cells within the tumor illustrated in **c. (d-f)** and **(g-i)** illustrate examples of Control peptide and Cripto BP grafts, respectively, showing no signs of these proliferating cell populations. **(j)** Pre-treatment of ESC with Cripto BP (BP), as well as BP infusion, markedly reduced tumor incidence upon transplantation compared to CP treatment. **(k)** Neural red stained sections showed that upon tumor occurrence, animals receiving Cripto BP pre-treated ESCs + BP infusion showed significantly reduced tumor volume by comparison to **(l)** Control Peptide. Ctx, cortex; CPu, caudate putamen; Lv, lateral ventricle.

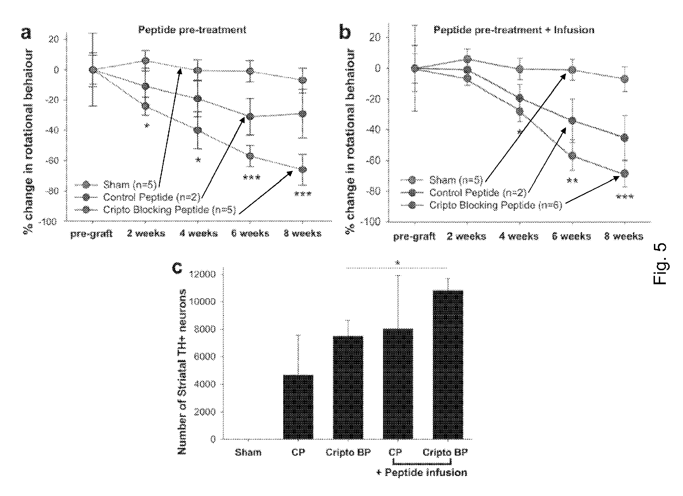

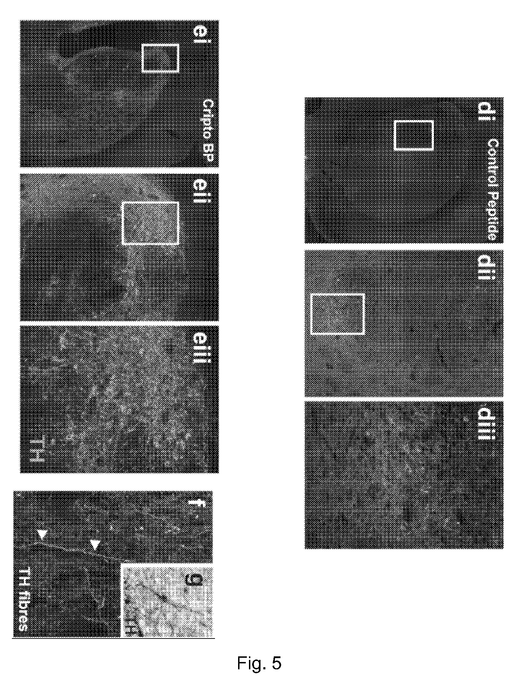

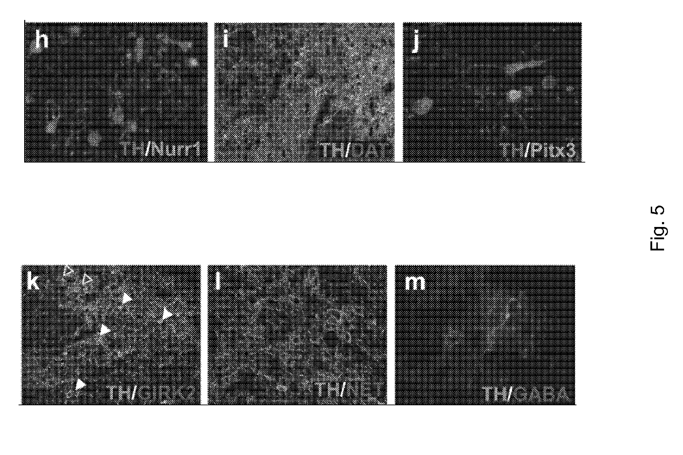

**Figure 5****.** Cripto BP induces behavioural and anatomical improvements in ESC-grafted Parkinsonian rats. **(a)** Pre-treatment of ESCs with Cripto BP or **(b)** Cripto BP pre-treated + BP peptide infusion (red line) significantly improves amphetamine-induced rotational behaviour of animals grafted, compared to sham grafting (green). **(c)** Number of TH-ir cells seen within the striatum following transplantation of ESCs treated with CP or Cripto BP (+ respective peptide infusion). *p<0.05 versus no peptide infusion. **(d)** Photomicrographs illustrating TH-ir neurons within the striatum following grafting of ESCs treated with CP + CP infusion, and **(e)** Cripto BP + BP infusion. **(f)** Numerous hypertrophied TH+ fibres seen outside the graft site, indicative of good graft integration. **(g)** High power image of a TH cell within the graft showing the bi-polar morphology characteristic of midbrain A9 DA neurons. **(h)** TH+/Nurr1+, **(l)** TH+/DAT+, **(j)** TH+/Pitx3+, **(k)** TH+/GIRK2 staining within the graft, confirming the midbrain dopaminergic phenotype of the TH neurons. **(l)** Norepinephrine (labelled with norepinephrine transporter, NET) fibres could be seen throughout the graft but did not co-localize with TH. TH neurons within the grafts did not co-localize with **(m)** GABA or, serotonin (5-HT, not shown). Data are mean ± SD. *p<0.05, **p<0.01, ***p<0.001, versus sham group.

### Materials and methods

### Synthesis of the combinatorial tetrameric tripeptide library

A tetrameric library composed of non-natural amino acids was prepared following methods reported in the recent literature. Commercially available 9-Fluorenylmethoxycarbonyl (Fmoc)-derivatized amino acids (purity >99%) and resins were purchased from Chem-Impex International (Wood Dale, IL, USA) and from Novabiochem (Laufelfingen, CH). Amino acids in the D configuration and others bearing TFA-stable side chain protections were chosen in order to increase the library chemical diversity and, at the same time, to introduce resistance to enzyme degradation for *in vitro* and *in vivo* applications (see **Table S1**).

**Table S1. List of building blocks used to assemble the peptide library. Building blocks 9, 20, 22, 23, 24, 25, 26, 27, and 28 bear side chain protection that are not removed by TFA treatment, so that unnatural residues are generated. The tosyl group on building block 20 stands for p-toluenesulfonate.**

| **code** | **3-letters code** | **building block** |
|---|---|---|
| 1 | D-Ala | D-Alanine |
| 2 | D-Asp | D-Aspartic acid |
| 3 | D-Val | D-Valine |
| 4 | D-Glu | D-Glutamic acid |
| 5 | L-Cha | L-Cyclahexylalanine |
| 6 | D-Phe | D-Phenylalanine |
| 7 | D-Thr | D-Threonine |
| 8 | D-Met | D-Methlonine |
| 9 | D-Cys(Acm) | D-Cysteine(S-acetamydomethyl) |
| 10 | D-Lys | D-Lysine |
| 11 | D-Tyr | D-Tyrasine |
| 12 | D-Pro | D-Proline |
| 13 | D-Leu | D-Leucine |
| 14 | D-His | D-Histidine |
| 15 | D-Gln | D-Glutamine |
| 16 | D-Trp | D-Triptophan |
| 17 | D-Arg | D-Arginine |
| 18 | D-Asn | D-Asparagine |
| 19 | D-Ile | D-Isoleudne |
| 20 | D-Arg(Tos) | D-Arginine(N^{r}-Tosyl) |
| 21 | D-Ser | D-serine |
| 22 | L-Cys(Acm) | L-Cysteine(S-acetamydomethyl) |
| 23 | L-Cys(Bzl) | L-Cysteine(S-benzyl) |
| 24 | L-Cys(p-MeBzl) | L-Cysteine(S-*p*methyl-benzyl) |
| 25 | L-Cys(tBu) | L-Cysteine(S-*tert*-butyl) |
| 26 | L-Met(O) | L-Methionine-sulphone |
| 27 | L-Met(O)₂ | L-Methionine-sulphoxide |
| 28 | L-Glu(β-OAII) | L-Glutamic acid-(β-allyl) |
| 29 | β-Ala | β-Alanine |
| 30 | Gly | Glycine |

The library was chemically synthesized following the Fmoc methodology ⁵³ and sequence randomization was achieved as reported elsewhere ^{38, 54}. In order to increase the molecular surface of the resulting peptides, a multimeric library was devised and prepared utilizing as common scaffold a polylysine core ⁴⁰. Two subsequent levels of dimerization achieved by using Fmoc-L-Lys(Fmoc)-OH as branching unit, allowed the generation of tetrameric structures onto which tripeptidic libraries were assembled. After resin cleavage, the libraries were submitted to repetitive cycles of TFA dissolution, precipitation and lyophilization to improve the overall quality of the final products. 30-components mixtures as well as single molecules were analyzed by LC-MS using an LCQ DECA XP mass spectrometer connected to a fully equipped Surveyor quaternary HPLC system (ThermoFisher, Milan, Italy). Biobasic C18 columns 50x2 mm ID RP18 were used for these analyses. Single molecules were prepared following the same Fmoc methodology and initially assayed as crude products. Purification to homogeneity was carried out by RP-HPLC on semi-preparative columns (LUNA 5x2,2 cm ID, RP 18, Phenomenex). Purity and identity of the purified peptides were assessed by LC-MS analysis. The dimeric 26-23-23 peptide was prepared in the same way, by performing one step of branching with Fmoc-L-Lys(Fmoc)-OH.

### Iterative deconvolution of the tetrameric tripeptide library.

To improve the overall solubility of the resulting mixtures, D-Glutamic (D-Glu), corresponding to building block 4 **(Table S1)** was initially chosen as the common, N-terminal residue. The total complexity of the library was therefore of 30² = 900 different peptides. In the first step of the deconvolution process, the peptide pools were tested with a molar excess of 30000-fold (calculated for each single peptide) over Cripto (8.6 x10⁻¹⁰ M; **Figure S1).** Once identified an active pool(s), a dose-dependent inhibition using 200-, 500-, 1000-, 5000-, 12500 and 30000 fold excess was performed to confirm the inhibitory property **(Figure S2).** The first active pool selected was that labelled as 4-23-X (X indicated random positions), where "23" identified the amino acid L-Cysteine(S-benzyl) [L-Cys(Bzl)] **(Figure S2).** This peptide pool was re-synthesized as 30 single peptides and submitted to the second screening using a molar excess of 1000-fold (calculated for each single peptide) over Cripto; the screening allowed the identification of the peptide 4-23-23 as the best performing in our test **(Figure S3).** To finally investigate the N-terminal position, where D-Glu was initially arbitrarily placed, 30 single peptides labelled as B-23-23 were synthesized and submitted to the final screening **(Figure S4, a).** Finally, a dose-dependent inhibition using 500-, 1000-, 2000-, 3000 and 4000 fold excess of the active pools was performed to confirm the inhibitory ability **(Figure S4, b).** The peptide indicated as 26-23-23, where "26" identified the amino acid L-Methionine-sulphoxide [L-Met(O)], was the molecule showing the optimal inhibitory activity. The activity of this peptide was further confirmed using the HPLC purified molecule.

### ELISA-based assays and screening of the combinatorial libraries

The screening was performed by an ELISA (Enzime-Linked Immunosorbent Assay) assay. Recombinant ALK-4-Fc chimeric protein (R&D) was coated on the surface of 96-well microtiter plates (100ng/well) and incubated over-night at Room Temperature (RT). After blocking with 1% BSA in PBS (180 µl/wells), the plate was incubated with in-house preparations of recombinant Cripto protein (⁵⁵; 20ng/well) in the presence of the indicated molar excess of each peptide pool, for 1 hr at RT followed by 1 hr at 37°C. Since the peptides were dissolved in DMSO, equimolar amounts of DMSO were added also to the control. Subsequently, the plate was incubated with rabbit polyclonal anti-Cripto antibodies (total IgG; 6µg/ml) for 1 hr at RT followed by 1 hr at 37°C. Following incubation with primary antibodies, Goat anti-rabbit HRP-conjugated IgG (Sigma Aldrich) were added diluted at 1:1000 to the plate and incubated for 1 hr at RT. Finally, the plate was developed with o-phenylendiammine peroxidase substrate (Sigma-Aldrich) and the absorbance was read at 490 nm on a microplate reader (Biorad Benchmark). The percentage of binding showed in presence of each pool, was evaluated by the following formula: % of binding = (OD^{490 nm} pool/ OD^{490 nm} Cripto100. Data were fitted using the software GraphPad Prism 4 (version 4.0, GraphPad software, Inc.) and the EC50 was derived using a nonlinear regression algorithm imposing a formula for one-site competition.

### Binding of 26-23-23 peptide (BP) to either Cripto or ALK-4 receptor by ELISA.

To evaluate the binding of Cripto BP to either Cripto or ALK-4 receptor, BP or control peptides (CP; 50µM) were coated on the surface of a 96-well microtiter plate and incubated over night at 4°C. Wells were washed five times with PBS Tween (PBT) and the aspecific sites blocked for 3 h at RT with 1 % Bovine Serum Albumin (BSA) in PBS, 180 µL/well. Wells were washed as above, and the recombinant form of either ALK-4-humanFc chimeric receptor or Cripto protein in PBT were added at the indicated concentrations and incubated for 1 h at 37°C followed by 1 hr at RT. After washing with PBT, to evaluate the binding of Cripto to 26-23-23, rabbit anti-Cripto IgG (6µg/ml) were added to the plates and incubated 1 h at 37°C and 1 hr at RT. After washing in PBT, a goat anti-rabbit HPRT (Santa Crutz) was added to the plate (0.3µg/ml) and incubated 1 h at RT. Finally, the plate was developed as reported above. To evaluate the binding of ALK4 to 26-23-23, the extracellular domain of ALK-4 receptor fused to human Fc (R&D Systems) was added at the indicated concentrations and a goat anti-human Fc (Jackson ImmunoResearch; 1:1000) was used.

### ESC culture and differentiation

Undifferentiated ESCs were cultured as previously described ⁵⁵. For *in vitro* differentiation as shown in Figure 2, ESCs were cultivated as embryoid bodies as described ³⁵_{.}

For more specific dopaminergic differentiation, R1 ESC were plated on a confluent monolayer of mitomycin-C treated (2 hour, 20ug/ml, Sigma) stromal PA6 cells (Riken Cell Bank) at a density of 100cells/cm² and differentiated based on the methods of Barberi et al, 2003 with the following modifications, **Fig. 3a** ²⁷. Cells were initially cultured in serum-replacement media (Dulbecco's modified Eagle medium with 15% serum replacement, 2mM L-glutamine and 10uM β-mercaptoethanol, Gibco-Invitrogen) containing Noggin (300ng/ml, R&D Systems). After 5 days the media was replenished and supplemented with Noggin plus morphogens fibroblast growth factor-8 (FGF8, 100ng/ml) and Sonic Hedgehog (Shh, 500ng/ml), previously shown to be important in midbrain DA neuron development. At day 8, cultures were changed into N2 media (a 1:1 mixture of F12 and MEM with 15mM HEPES buffer, 1 mM glutamine, 6mg/ml glucose (Sigma-Aldrich), 3mg/ml Albumax and N2 supplement (all purchased from Invitrogen)) supplemented with FGF2 (20ng/ml), Shh, FGF8 and Wnt5a (previously shown to be important in the differentiation of dopaminergic precursors *in vitro* and *in vivo* ^{25, 56}. Finally at day 11, cultures were exposed to survival factors brain derived neurotrophic factor (BDNF, 30ng/ml), glial derived neurotrophic factor (GDNF, 30ng/ml) and ascorbic acid (AA, 100uM). In addition, peptides (Control peptide, CP; Dimer peptide, DP or Cripto Blocking peptide, Cripto BP, 12.5uM) were added to the cultures from day 1-11. Peptides were replenished in the media every second day.

### Western blotting and Smad2 phosphorylation assay

Two day-old *cripto^{-l-}* Embryoid Bodies (EBs) were serum starved for 3 hrs in DMEM (GIBCO) without LIF and in low serum (0.5% FBS). Following starvation, EBs were incubated for 20 min at 37°C with recombinant Cripto protein (0.5og/ml) either alone or in the presence of the indicated amount of peptides. Finally, EBs were dissolved in Laemmli lysis buffer and analyzed by Western blot using the Trans-Blot Semi-dry System (BIO-RAD), following manufacturer's instruction. Anti-Smad2 and anti-phospho-Smad2 (Ser465/467) Antibodies (Cell Signalling Technology) were used, following the manufacturer's instructions.

### RNA preparation and RT-PCR

Total RNAs from 13 day -old EBs was extracted with TRlzol kit (Life Technologies Inc.) according to manufacturer's instructions. One microgram of total RNA was used for cDNA synthesis with SuperScript II reverse transcriptase (Life Technologies Inc.) and random hexamers. Quantitative Real time PCR was performed using SYBR Green PCR master mix (EuroClone), according to manufacturer's instructions. The primers used are described in **Table S2.**

**Table S2. List of primers used for QRT-PCR analysis.**

| **Gene** | **Cycles** | **Primer (sense)** | **Primer (antisense)** | **Product size (bp)** |
|---|---|---|---|---|
| MLC2a | 28 | cagacctgaaggagcctattccc | gtcagcgtaaacagttgctctacc | 300 |
| α-foetoprotein | 30 | ccacgttagattcclcccagtgcgt | catacttgttagagagttccgtctc | 200 |
| NFM | 32 | gaaatggaagaaaccctcaca | ccggccttggcctctggttttgg | 474 |
| TH | 30 | tgtcacgtccccaaggttcat | gggcaggccgggtctctaagt | 276 |
| DAT | 30 | gtgggcttcactgtcatcctca | cccaggtcatcaatgccacga | 514 |
| Wnt1 | 30 | acctgttgacggattccaag | tcatgaggaagcgtaggtcc | 462 |
| β-tubulin | 28 | gggaggtgataagcgatgaa | cccaggttctagatccacca | 150 |

### Determination of in vitro dopamine and dihydroxphenylacetic acid (DOPAC) levels

Dopamine release and turnover was determined *in vitro* using reverse phase high performance liquid chromatography (HPLC). Mouse ESC were differentiated in a 12 well plate according to the methodology depicted in figure 3A, in the presence of either the Control peptide of Cripto BP. After 14days in vitro, media was replaced with 200ul of N2 media supplemented with 56mM KCI for 30min. The media was then collected and stabilized with 20ul of buffer containing 0.4 M perchloric acid containing 7.9mM sodium metabisulphate and 1.34mM disodium EDTA. On the day of analysis the samples were centrifuged and the supernatant transferred to HPLC vials and placed in an autosampler for injection onto the HPLC. The HPLC consisted of a LC-20AT pump (Shimadzu), SIL-20A Autosampler (Shimadzu) and C18 reverse phase column (Bio-Rad, Hercules, USA). Detection was via a 3mm VT-03 flow cell with glassy carbon working electrode (Antec Leyden) and Decade II Electrochemical Detector (Antec Leyden). The mobile phase consisted of 17% v/v methanol in purified deionized water containing 70mM KH2PO4, 0.5mM EDTA and 8.0mM sulfonic acid, pH 3.0 and was run at a flow rate of 0.5ml/min. DA turnover was expressed as the concentration of DOPAC to DA [DOPAC/DA]. Analysis was performed on triplicate wells on three independent differentiation experiments.

### 6-OHDA lesioning and Rotational Behavior

Thirty-seven adult male Sprague Dawley rats (250-350g; from ARC, Australia) were housed and treated according to the guidelines of the Australian and Howard Florey Institute animal ethics committee (07-030). Animals received unilateral stereotaxic injections of 6-OHDA into the substantia nigra pars compacta (SNpc) to create a complete lesion of the midbrain DA neurons, as previously described ²⁹. Lesioned animals were selected for transplantation based on their response to amphetamine induced rotational behaviour as previously described ²⁹. Animals making greater than 6 rotations/min (lesion greater than 80%) were selected for grafting.

### Transplantation of ESCs

Two days prior to grafting animals began cyclosporine-A immune suppression (10mg/kg). Fourteen days post-lesioning selected animals (determined by behavioural rotations) were again anesthetized and stereotaxically injected with 1µl of cell suspension at each of the 4 sites as previously described ²⁹. Stereotaxic coordinates of 1^{st} & 2^{nd} Injection (from bregma): anterior 0.2mm, lateral 3.0mm, ventral 4.5 and 5.0mm; 3^{rd} & 4^{th} Injection, anterior 1.0mm, lateral 2.5mm, ventral 4.5 and 5.0mm; incisor bar 0mm ⁵⁷). ESCs were differentiated as outlined in **Fig. 3a****.** In brief, ESC colonies were dissected away from the stromal feeder layer and dissociated using collagenase/dispase with agitation (700µg/ml, Roche, for 20min, 80rpm). Cells were subsequently resuspended at a density of 3,000 cells/µl ready for grafting. Five rats received Sham injections (4µl N2 media,1µl/site), 16 rats received a total of 12,000 ESCs (4x 1µl grafts) pre-treated with the Control peptide (of which 9 of these animals also received infusion of the Control peptide) and a further 16 rats received grafts pretreated with the Cripto blocking peptide (9 animals receiving infusion of the Cripto BP).

The peptide infusion was performed by implantation of a cannula into the striatum (Alzet brain infusion kit) connected to a subcutaneously placed osmotic minipump (Alzet) prefilled with either CP or Cripto BP. Implantation was performed at the same time as the cell grafting. Cannula coordinates: anterior 0.5mm, lateral 1.5mm, ventral 5.0mm; incisor bar 0mm ⁵⁷. The osmotic pump was capable of continuously delivering the peptide over a 3 weeks period (75µM, 0.25µl/hr) upon which time the animals were briefly anaesthetized with isoflurane and the pump removed through a small incision over the scapular region.

Eight weeks post grafting, rats were killed by an overdose of sodium pentobarbital (100 mg kg⁻¹ i.p.) and intracardially perfused as previously described ²⁹. Brains were removed, post fixed for 1 hour, and then left overnight at 4°C in 20% sucrose in PBS. The following day, 20µm-thick coronal sections were cut serially through the striatum (12 series) and 50µm through the SNpc and mounted directly onto chrome alum [K₂Cr(SO₄)₂·12H₂O] gelatinized slides.

### Histological analysis

Immunofluorescence analysis on adherent EBs, dopaminergic differentiated ESC and striatal grafts were performed as previously described ^{29, 35}.. The following primary antibodies were used: mouse anti-βIII-tubulin (Tuj1, 1:1000, Promega); anti-βIII tubulin (Sigma; 1:400), rabbit anti-tyrosine hydroxylase (TH, 1:200, PelFreez); mouse anti-TH (1:500, Chemicon); Nurr-1 (1:250, Santa Cruz); rabbit anti-Pitx3 (1:200, InVitrogen), rabbit anti-GIRK-2 (1:100, Chemicon); rat anti-DAT (1:1000, Chemicon); rabbit anti-5HT (1:200, Biogenesis); rabbit anti-NET (1:20, Chemicon), rabbit anti-GABA (1:500, Sigma); rabbit anti-Ki67 (1:1000, Neomarkers); and rat anti-Nestin (1:100, Developmental Studies Hybridoma Bank). Appropriate fluorophore conjugated secondary antibodies were used for visualization: (Cyanin-2, Cy3 and Cy5, 1:500, Jackson Immunoresearch or Alexa-488, Alexa-594 and Alexa-633, 1:200, Molecular Probes).

Hoescht counterstaining was used for visualization of all cells within the cultures. The proportion of neuron containing colonies (labeled for using the immature neuronal marker βIII-tubulin (Tuj1)) and the number of dopaminergic neurons/µm² colony (identified using a marker for the precursor enzyme in DA synthesis,TH) were counted. Differentiation experiments were performed in triplicate.

One series of slides containing grafted striata was counterstained using 1% Neutral red to visualise the graft/tumor size and the gross morphology of the striatum. Labeling was visualized by light or fluorescent illumination using an inverted microscope (DMIRB, Leica Microsystems, Wetzelar, DE, http://www.leicamicrosystems.com) or upright microscope (Leica DMLB2). Images were acquired on a DC 350 FX camera (Leica). Area of grafts and tumors was calculated using Stereo Investigator software (MicroBrightField, VT, USA).

### Statistical analysis

Values are expressed as means SEM or SD. The statistical significance of difference was determined by ANOVA followed by Tukey's test; except for data in Figure 5 (Student's t-test).

### Results

### Identification of a novel peptide that antagonizes CriptolALK-4 receptor interaction.

In order to identify Cripto/ALK-4 antagonists capable of disrupting downstream signalling, the authors screened a tetrameric tripeptide library synthesized using 29 non-natural amino acids to increase peptide stability, plus Glycine, (see **Table S1**). To increase the molecular surface, the library was assembled as previously described ³⁸ on a peptide scaffold composed by a poly-lysine core ⁴⁰. In order to facilitate the characterization of the peptide mixtures, and at the same time to expedite the screening procedure, the first residue was arbitrarily fixed. A negatively charged amino acid, the D-Glutamic acid (D-Glu, denoted 4; **Fig. 1a** and **Table S1**), was thus initially chosen as the common, N-terminal residue, to favour peptide solubilisation. The library was arranged in 30 pools, each of them composed by 30 tetrameric tripeptides, which differ for the amino acid (denoted B) on the second position **(****Fig. 1a****),** while the residue in the third position (denoted X) was randomly added. The total complexity of the library was therefore of 30²=900 different peptides. The screening was carried out by an ELISA-based competition assay whereby the displacement of soluble recombinant Cripto from coated ALK-4 by the peptide pools was evaluated. The iterative procedure consisted of three screening rounds, which led to the selection of the 26-23-23 tetramer formed by L-Methionine-sulphoxide [L-Met(O)] (denoted as 26) in position 1, and S-benzylated L-Cysteine [Cys(Bzl)] (denoted as 23) in positions 2 and 3 of the tripeptide **(****Fig. 1a****),** (for details see Material and methods and **Figs S1-S4**). The selected tetrameric tripeptide (26-23-23) was thereafter named Cripto Blocking Peptide (BP).

Below is the schematic representation and chemical structure of the 26-23-23 tetrameric tripeptide (Cripto BP) that has a calculated MW of 2536.5 amu (atomic mass unit, average value).

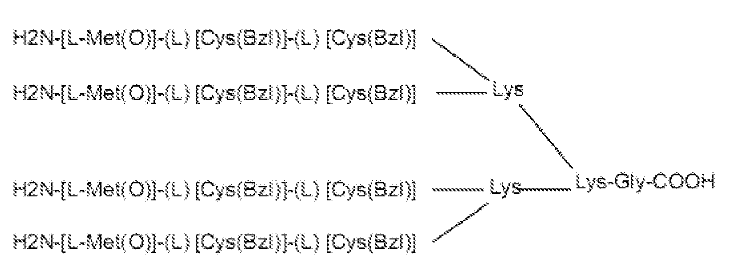

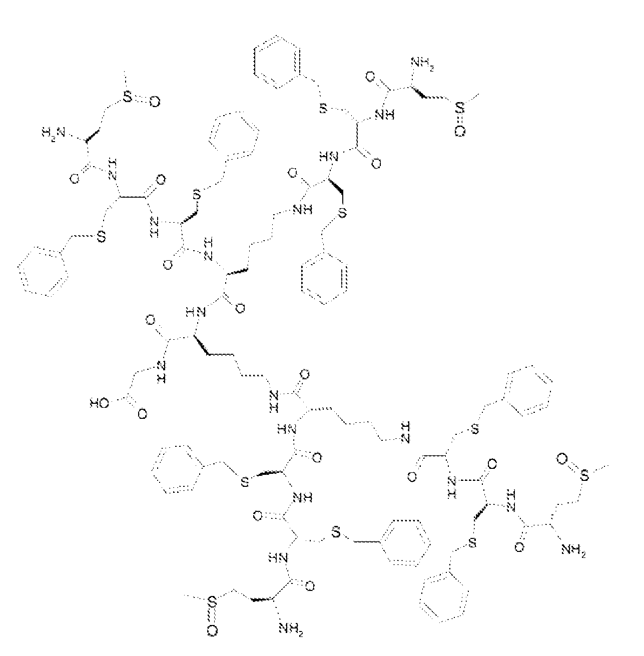

This peptide blocked the interaction of Cripto and ALK-4 with an estimated IC₅₀ of 400nM, while the Control tetrameric Peptide D-Ser-Ala-Cha (CP) and a Dimeric Peptide variant (DP) of the 26-23-23 peptide had no effect **(****Fig. 1b****).** The authors thus performed ELISA assays to evaluate whether Cripto BP- or CP-coated to microtiter plates could bind recombinant Cripto and/or ALK-4 receptor. Cripto but not ALK-4 specifically recognized the immobilized Cripto BP, in a dose-dependent manner **(****Fig. 1c****)**. Neither Cripto nor ALK-4 recognized the control peptide, confirming the specificity of Cripto BP for Cripto binding.

### Cripto BP antagonizes Cripto signalling and impairs cardiomyogenesis in ESC cultures

Given the neutralizing activity of the peptide, the authors then asked whether Cripto BP was able to block ALK-4/Smad2 -dependent Cripto signalling in ESCs and thereby mimic the absence of *cripto* in an ESC differentiation assay. The authors first evaluated whether Cripto BP was able to prevent Cripto-induced Smad2 phosphorylation of serum starved 2 day-old *cripto^{-l-}* Embryoid Bodies (EBs), as assessed by Western blot analysis. Recombinant soluble Cripto protein stimulated Smad2 phosphorylation³⁵. This effect was not modified by the presence of increasing amounts of either Cripto DP or CP (2.5µM and 25µM) **(****Fig. 2a****).** On the contrary, Cripto-induced Smad2 phosphorylation was inhibited by Cripto BP and completely blocked at 25µM (125x molar excess; **Fig. 2a**). The authors thus asked whether the neutralizing activity of Cripto BP on endogenous Cripto signalling was also able to block cardiomyogenesis in wild-type ESCs. To this end, 2 day-old wild-type EBs were treated with increasing amounts of either CP or Cripto BP, ranging from 5.0 to 25□M, and the percentage of EBs containing beating areas was scored from days 8 to 12 of the differentiation (n=∼60 EBs/group; **Fig. 2b**). Given that the peptides were dissolved in DMSO, control untreated EBs were compared with DMSO treated EBs. Interestingly, neither DMSO, the tetrameric CP **(****Fig. 2b****)**, nor the Dimeric Peptide (DP, data not shown) affected the percentage of beating EBs. Instead, treatment of wild-type EBs with Cripto BP, induced a dose-dependent inhibition of cardiomyogenesis, as shown by the progressive reduction of rhythmically contracting EBs (at 25□M, BP 3.7% ± 3.5 vs. CP 82.9% ± 11.17, **p<0.0001) **(****Fig. 2b****).**

### Cripto BP promotes neuronal differentiation of wild-type ESCs

The authors previously showed that *cripto-l-* ESCs spontaneously differentiate into neurons during the time window when wild-type ESCs are primed for cardiomyogenesis by Cripto ³⁵. Thus, if specific and effective, Cripto BP should be able to re-direct ESC fate and promote neural induction and neurogenesis. To address this issue, 2 day-old wild-type EBs were treated as described above, with either control (CP), dimeric (DP) or blocking (BP) peptides and both morphological and immunofluorescence analysis were performed on 13 day-old EBs. Anti-βIII tubulin antibodies were used to recognize the neuron-specific form of class III β-tubulin. To better define the activity of the peptides, the authors arbitrarily defined 4 grades of neuronal differentiation ranging from the absence of neurons (Grade 0) to full neuronal differentiation (Grade 3), i.e. presence of a dense network of βIII-tubulin -positive cells. The presence of either few isolated neurons or small areas of βIII-tubulin positive cells defined intermediate phenotypes, Grade 1 and Grade 2, respectively **(****Fig. 2c****)**. Our data clearly indicated that Cripto BP but not CP was able to efficiently induce neuronal differentiation, characterised by a high percentage of Grade 3 phenotype (*BP* 45.6% ± 5.2 p<0.001 vs *CP* 6.9% ± 1.15 p<0.005) **(****Fig. 2d****).** Accordingly, upon treatment with control peptides (CP or DP), the majority of the EBs scored showed a Grade 0 phenotype (*CP* 75.9% ± 7.02 p<0.003; *DP* 77.2% ± 6.9 p<0.001; *BP* 17.4% ± 1.15 p<0.001; **Fig. 2d**); thus confirming the specific effect of Cripto BP. To support these findings, the authors examined the expression profile of both cardiac and neuronal differentiation markers in the same experimental settings by QRT-PCR. Similar to our previous findings in *cripto* -*l*- ESCs²⁹, expression of the pan neuronal marker NFM was induced in 13 day -old EBs upon treatment with Cripto BP, but not by the control peptide. Concomitantly, expression of the cardiac specific marker MLc2v was reduced **(****Fig. 2e****)**. Thus, our results suggest a fate switch in ESCs from cardiomyocytes to neurons. Moreover, similar to *cripto* -*l*- ESCs ³⁷, Cripto BP -treated wild-type EBs also showed expression of markers characteristic of developing midbrain dopaminergic neurons. These comprise the expression of factors important for early stages of dopaminergic neuron development including Lmx1a ²¹ and Wnt1 ^{22,33} (data not shown), as well as late markers of DA differentiation such as Pitx3 ⁴¹, Wnt5a ^{25,34}, Shh ^{16,42}, TH (the rate limiting enzyme in DA synthesis) and DAT (dopamine transporter; **Fig. 2e**).

### Increased dopaminergic differentiation of ESCs treated with Cripto BP

The authors next examined whether DA differentiation could be promoted. To this end, the authors used a DA differentiation protocol involving co-culturing on stromal cells ²⁷, to which additional modifications were incorporated **(****Fig. 3a****).** In brief, ESCs were plated at low density onto stromal cells (PA6) for a period of 5 days, to promote neural induction. From day 0-8, cultures were treated with noggin to enhance neuronal induction. From day 8-11, cultures where changed into neuronal (N2) media in the presence of Shh and FGF-8 (for ventral midbrain patterning) and basic FGF (to expand the pool of DA precursors), as well as Wnt5a (involved in DA neuron differentiation *in vitro* and *in vivo* ^{25,43}). Control peptide (CP) or Cripto BP (BP) were added to the differentiating ESCs from day 1 to day 11. Finally (days 11-14), the cells were exposed to the survival factors BDNF, GDNF and ascorbic acid (AA) **(****Fig. 3a****).** Following differentiation in the presence of the Cripto BP, the majority of colonies within the cultures were immunoreactive for βIII-tubulin (91% compared to 66% in control peptide, **Fig. 3b**). Furthermore, Cripto BP was capable of significantly increasing the proportion of colonies containing TH-ir DA neurons compared to CP (62% and 34%, respectively, **Fig. 3c**). Additionally, these TH-ir colonies contained significantly more DA neurons (TH+ cells/om colony, 286% greater than control, **Fig. 3d****-f**). The yield of DA neurons derived from wildtype ESCs was enhanced further by differentiation in the presence of Noggin and Wnt5a, in conjunction with Cripto BP **(****Fig. 3c****-d)**. Many of the TH labelled neurons showed bipolar morphology (**arrow,** **Fig. 3f****"**), a characteristic feature of the A9 ventral midbrain DA neurons that are lost in PD.

Immunohistochemistry for GIRK2, preferentially expressed in SNpc DA neurons ⁴⁴, Nurr1 and Pitx3 confirmed that TH-ir cells within the Cripto BP cultures were of a midbrain DA phenotype **(****Fig. 3g****-i)**. Interestingly, DA differentiation increased at the expense of other neuronal subpopulations; as observed by the low numbers of γ-aminobutyric acid (GABA-ir) and serotonin (5HT-ir) neurons present in the culture **(****Fig. 3j****-k)**. Furthermore, the authors analysed the ability of the dopamine cells generated *in vitro* to synthesise, release and utilize dopamine. Whilst both Cripto BP and CP treated cultures were able to utilize DA (as reflected by dopamine turnover, ratio of DOPAC to DA, **Fig3l"'****)**, the authors noted that Cripto BP treatment of ESCs significantly increases the KCI-evoked release of dopamine **(****Fig3l'****)** and DOPAC **(****Fig3l"****)** within the cultures, compared to Control peptide treatment - reflective of the enhanced DA differentiation.

The authors next examined the ability of ES-derived DA neurons to survive, integrate and induce functional recovery *in vivo,* after transplantation in animal models of Parkinson's disease.

### Reduced tumor formation and enhanced functional recovery in Parkinsonian rats receiving ESC grafts treated with Cripto BP

The effects of ESC grafts pre-treated *in vitro* with Cripto BP or CP, or pre-treated and infused with the same peptides, were examined in Parkinsonian rats. The validity of the model was assessed by amphetamine-induced rotational behaviour and histological examination, which confirmed that all animals had near or complete unilateral lesions of SNpc DA neurons (cell loss >90% and >6 rotations/min; data not shown). Additionally, the viability of the grafts was also examined; 97% of the animals transplanted with 12,000 ESC-derived cells showed viable grafts at 8 weeks. The authors first examined the presence of tumors within the grafts, as assessed by the presence of cell masses containing proliferating (Ki67+) and undifferentiated neural stem/progenitor cells (Nestin+) within the striatum **(****Fig. 4a****-c)**. A substantial decline in tumor formation was detected in rats receiving ESCs pre-treated with Cripto BP (42%) compared to CP (71%; **Fig. 4j**). Please note that additional manipulations that tend to decrease tumor formation, such as high-density cultivation prior to transplantation, were not performed. However, the authors did examine whether continual Cripto BP infusion into the striatum would affect tumor incidence, and found a further 9% reduction **(****Fig. 4j****).** Examples of grafts, which did not show proliferating or undifferentiated cells are shown in Figure **4d****-f** and **4g-i.** These represented 25%-29% of the CP-treated and 58-67% of the Cripto BP-treated animals. Thus, the authors' results illustrate that Cripto BP reduces tumor formation by more than 50% compared to control peptide. Interestingly, when tumor volume was examined, animals receiving ESCs pre-treated with Cripto BP showed a marked reduction (1.09um³ X 10¹⁰ ± 0.58 X 10¹⁰) compared to Control peptide (2.34um³ X 10¹⁰ ± 0.60 X 10¹⁰, Mean ± SEM). Furthermore, Cripto BP infusion following transplantation significantly reduced the volume of tumors generated by Cripto BP-pretreated ESCs (0.79um³ X 10¹⁰ ± 0.16 X 10¹⁰), as assessed in neural red stained sections compared to Control peptide (*p<0.05, **Fig. 4k-1****, Table 1**)**.**

**Table1: Volume of tumors following transplantation of ESC pre-treated with peptides, as well as transplantation of ESC pre-treatment peptides + peptide infusion. ANOVA with Tukey post-hoc test, *p<0.05.**

| Total Striatal Volume: 3.17 x 10₁₀ + 0.06 x 10₁₀ | |
|---|---|
| | **Tumor Area (um3)** |
| **Control Peptide** | 2.34 x 10₁₀ + 0.60 x 10₁₀ |
| **Cripto BP** | 1.09 x 10₁₀ + 0.58 x 10₁₀ |
| **Control Peptide** (+ **CP infusion)** | 2.05 x 10₁₀ + 0.76 x 10₁₀ |
| **Cripto BP** (+ **BP infusion)** | 0.79 x 10 ₁₀ + 0.16 x 10₁₀ |

The authors next examined the impact of ESC transplantation on amphetamine-induced circling behaviour and dopamine neuron engraftment in rats that did not present any tumor. Note that due to the high incidence of tumors (more than 70%) in the Control peptide conditions, only 2 animals remained in the Control peptide treatment and control peptide treatment+ infusion, precluding thus statistical analysis. Functionally, the motor behaviour of Parkinsonian rats receiving ESCs grafts pre-treated with Cripto BP, without infusion **(****Fig. 5a****),** or with infusion **(****Fig. 5b****),** significantly improved compared to Sham treated animals. Grafting of 12,000 ESCs pre-treated with Control peptide showed modest levels of functional improvement compared to shams. However, animals receiving the same number of ESCs pre-treated with the Cripto BP showed significant reductions in amphetamine induced rotational behaviour as early as week 2 post-grafting. Moreover, the reduction in motor asymmetry progressively improved until 8 weeks postgrafting (Cripto BP: 66.2 ±8.4% reduction, compared to sham: 7+11%, mean + SD, **Fig. 5a**). While Control peptide treated and infused grafts induced a modest improvement in amphetamine-induced rotations, treatment of ESCs with Cripto BP + infusion significantly improved functional recovery in PD rats compared to sham grafted animals (sham: 7± 8% improvement, Cripto BP: 69.6 ± 8.4%, mean ± SD, **Fig. 5b**).

The authors next confirmed that the functional improvement observed in Cripto BP treated animals correlated with TH-ir neuron counts within the grafts in tumor-free animals. No TH neurons were noted within the striatum of animals receiving sham grafts. Animals grafted with Cripto BP treated ESCs showed more TH+ cells compared to Control peptide treated ESCs (7491 ± 1488, n=5; and 4688 ± 3047, n=2, respectively). Similarly, animals receiving ESC grafts pre-treated and infused with Control peptide showed less TH+ cell counts (8045 ± 4095, n=2) than Cripto BP treated and infused grafts. Interestingly, this condition showed a consistent and significant increase in TH-ir cell counts (10815 ± 887) compared to BP pre-treatment alone, illustrating that the infusion of Cripto BP further improves the engraftment of ESC-derived DA neurons pre-treated with Cripto BP **(****Fig. 5c****-e)**. Many of the cells within the grafts displayed classical bipolar morphology **(****Fig. 5g****)** as well as extensive hypertrophied neurites throughout the striatum **(****Fig. 5f****).** Immunohistochemistry revealed that the many of the TH-ir cells within the graft also co-expressed Nurr1, DAT, Pitx3 and GIRK2, verifying their midbrain DA identity **(****Fig. 5h****-k)**. Interestingly, Pitx3/TH and GIRK2/TH co-labeling illustrated that the majority of TH neurons within the graft were in fact SNpc midbrain-like DA neurons (white arrowheads in **Fig. 5k**). Whilst a number of norepinephrine fibres (labelled with norepinephrine transporter, NET) could be see within the grafts **(****Fig. 5l****),** no TH-ir cell bodies within the graft co-localised with other neurotransmitters, including norepinephrine **(****Fig. 5l****),** GABA (**Fig. 5m**) and serotonin (not shown).

Results show that a synthetic peptide, Cripto BP, reliably reduces tumor formation, improves the generation of midbrain DA neurons from ESCs and results in a net behavioural improvement in Parkinsonian rats.

### Bibliography

1. Ding, S. & Schultz, P.G. Nat Biotechnol 22, 833-840 (2004).
2. Chen, S. et al. Proc Natl Acad Sci U S A 103, 17266-17271 (2006).
3. Huangfu, D. et al. Nat Biotechnol 26, 1269-1275 (2008).
4. Desbordes, S.C. et al. Cell Stem Cell 2, 602-612 (2008).
5. Lindvall, O. & Kokaia, Z. Nature 441, 1094-1096 (2006).
6. Parish, C.L. & Arenas, E. Neurodegener Dis 4, 339-347 (2007).
7. McKay, R. & Kittappa, R. Neuron 58, 659-661 (2008).
8. Ho, H.Y. & Li, M. Regen Med 1, 175-182 (2006).
9. Lindvall, O. & Hagell, P. Prog Brain Res 127, 299-320 (2000).
10. Mendez, I. et al. Nat Med 14, 507-509 (2008).
11. Winkler, C., Kirik, D. & Bjorklund, A. Trends Neurosci 28, 86-92 (2005).
12. Lee, S.H., et al., Nat Biotechnol 18, 675-679. (2000).
13. Kim, J.H. et al. Nature 418, 50-56 (2002).
14. Sanchez-Pernaute, R. et al. Brain 131, 2127-2139 (2008).
15. Danielian, P.S. & McMahon, A.P. Nature 383, 332-334 (1996).
16. Ye, W., et al., Cell 93, 755-766 (1998).
17. Zetterstrom, R.H. et al. Science 276, 248-250 (1997).
18. Smidt, M.P. et al. Nat Neurosci 3, 337-341 (2000).
19. Nunes, I., et al., Proc Natl Acad Sci U S A 100, 4245-4250 (2003).
20. Kele, J. et al. Development 133, 495-505 (2006).
21. Andersson, E. et al. Cell 124, 393-405 (2006).
22. Puelles, E. et al. Development 131, 2037-2048 (2004).
23. Joksimovic, M. et al. Nat Neurosci 12, 125-131 (2009).
24. Kittappa, R., et al., PLoS Biol 5, e325 (2007).
25. Castelo-Branco, G. et al. Proc Natl Acad Sci U S A 100, 12747-12752 (2003).
26. Andersson, E.R. et al. PLoS ONE 3, e3517 (2008).
27. Barberi, T. et al. Nat Biotechnol 21, 1200-1207 (2003).
28. Perrier, A.L. et al. Proc Natl Acad Sci U S A 101, 12543-12548 (2004).
29. Parish, C.L., et al., Stem Cells 23, 471-476 (2005).
30. Reissmann, E. et al. Genes Dev 15, 2010-2022 (2001).
31. Cheng, S.K., et al Genes Dev 17, 31-36 (2003).
32. Schier, A.F. Curr Biol 13, R192-194 (2003).
33. Massague, J. & Chen, Y.G. Genes Dev 14, 627-644 (2000).
34. Gray, P.C., Proc Natl Acad Sci U S A 100, 5193-5198 (2003).
35. Parisi, S. et al. J Cell Biol 163, 303-314 (2003).
36. Gray, P.C., et al., Mol Cell Biol 26, 9268-9278 (2006).
37. Shani, G. et al. Mol Cell Biol 28, 666-677 (2008).
38. Marino, M., et al., Nat Biotechnol 18, 735-739 (2000).
39. Ponticelli, S. et al. J Biol Chem 283, 34250-34259 (2008).
40. Tam, J.P. Proc Natl Acad Sci U S A 85, 5409-5413 (1988).
41. Smidt, M.P., Smits, S.M. & Burbach, J.P. Cell Tissue Res 318, 35-43 (2004).
42. Roussa, E., Farkas, L.M. & Krieglstein, K. Neurobiol Dis 16, 300-310 (2004).
43. Parish, C.L. et al. J Clin Invest 118, 149-160 (2008).
44. Schein, J.C., Hunter, D.D. & Roffler-Tarlov, S. Dev Biol 204, 432-450 (1998).
45. Bjorklund, L.M. et al. Proc Natl Acad Sci U S A 99, 2344-2349 (2002).
46. Snyder, B.J. & Olanow, C.W. Curr Opin Neurol 18, 376-385 (2005).
47. Yoshizaki, T. et al. Neurosci Lett 363, 33-37 (2004).
48. Chung, S. et al. J Neurochem 97, 1467-1480 (2006).
49. Hedlund, E. et al. Stem Cells 26, 1526-1536 (2008).
50. Huangfu, D. et al. Nat Biotechnol (2008).
51. Minchiotti, G. Oncogene 24, 5668-5675 (2005).
52. Schmierer, B. & Hill, C.S. Nat Rev Mol Cell Biol 8, 970-982 (2007).
53. Fields, G.B. & Noble, R.L. Int J Pept Protein Res 35, 161-214 (1990).
54. Furka, A., et al., Int J Pept Protein Res 37, 487-493 (1991).
55. Minchiotti, G. et al. Development 128, 4501-4510 (2001).
56. Parish, C.L. et al. J Clin Invest 118, 149-160 (2008).
57. Paxinos, G. & Watson, C. The rat brain in stereotaxic co-ordinates, Edn. 3rd. (Academic Press, Sydney; 1998).

## Claims

1. A tripeptide molecule of formula (I):
Y₁-(X1)-(X2)- (X3) (I)
wherein
Y₁ is an amino-terminal group or an acetylated amino-terminal group;
X1 belongs to the group of (R)- or (S)-Glu, (R)- or (S)-Met, (R)- or (S)-Met[O], (R)- or (S)-Met[O]₂, where Glu is glutamic acid, Met is methionine, Met[O] is methionine sulphoxide, Met[O]₂ is methionine sulphone, (R)- or (S)- refer to the R or S absolute configuration;
X2 and X3 are an (R)- or (S)-(S-aryl)-Cysteine, where S-aryl means any aryl group directly linked to the thiol group of the cysteine; (R)- or (S)- indicates the absolute configuration on the chiral center of the cysteine; and are equal or different;
or any combination thereof.

2. A tripeptide molecule of formula (I) according to claim 1 wherein X1 is S-Met[O], R-Glu or S-Met[O]₂, X2 and X3 are S-Cys(Bzl) (S-benzylcysteine), or any combination thereof.

3. A multimeric branched tripeptide molecule of formula I according to any of previous claims wherein each tripeptide is attached to a branching scaffold.

4. The multimeric branched tripeptide molecule according to claim 3 comprising from 2 to 8 of said tripeptides.

5. The multimeric branched tripeptide molecule according to claims 3 or 4, wherein the branching scaffold has the formula (II):
Z-Y₂ (II)
wherein
Z consists of one or more trifunctional amino acids (TFAA), being equal or different each other;
Y₂ is a hydroxyl- or amino group, an amino acid, or a peptide linked via an amide bond to the terminal carbonyl group of Z;
Y₂ is equal or different from the tripeptide of formula (I).

6. The multimeric branched tripeptide molecule according to claim 5 wherein the TFAA comprises as functional groups two amino groups and one carboxyl group.

7. The multimeric branched tripeptide molecule according to any of claims 3 to 6 wherein each tripeptide of formula (I) is linked to an amino group of said TFAA.

8. The multimeric branched tripeptide molecule according to any of claims 5 to 7 wherein said trifunctional amino acid (TFAA) has the formula (III): 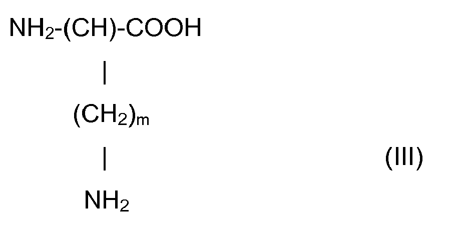 wherein
m is an integer from 1 to 4; the amino acid is a R- or S-enantiomer.

9. The multimeric branched tripeptide molecule according to any of claims 5 to 8 wherein Z consists of 3 or 7 TFAAs, forming a (TFAA)₂-TFAA- or (TFAA)₄-(TFAA)₂-TFAA-branching scaffold, respectively.

10. The multimeric branched tripeptide molecule according to any of claims 5 to 9 wherein said said TFAAs are R- or S-enantiomers of 2,3-diaminopropionic acid, of 2,4-diaminobutyric acid, of ornithine, of 2,5-diaminopentanoic acid, of lysine, or of 2,6-diaminoesanoic acid.

11. The multimeric branched tripeptide molecule according to any of claims 5 to 10 wherein Y₂ is a small or a non-bulky amino acid, such as glycine or alanine; or tripeptide formed of any combination of R-arginine, S-arginine, R-lysine, S-lysine.

12. The multimeric tripeptide molecule according to claims 5 to 11 of formula 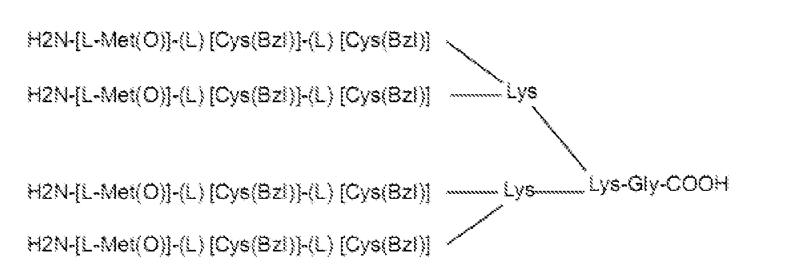 wherein [L-Met(O)] is L-Methionine-sulphoxide and [Cys(Bzl)] is S-benzylated L-Cysteine.

13. Use of the molecule according to any of previous claims for specifically and selectively binding to Cripto protein and acting as a competitor for the binding of Cripto protein to the Alk-4 receptor and as inhibitor of Cripto-dependent Smad2 phosphorylation.

14. Use of the molecule according to claims 1 to 12 for selectively inhibiting the Cripto-dependent differentiation process of stem cells, determining the induction of stem cell differentiation into neuronal cells.

15. Neuronal induced stem cells treated with the molecule according to claim 14.

16. The molecule according to claims 1 to 12 or the neuronal induced stem cells according to claim 14 for medical use.

17. The molecule according to claims 1 to 12 or the neuronal induced stem cells according to claim 15 for the treatment of a neurodegenerative disease.

18. The molecule according to claims 1 to 12 or the neuronal induced stem cells according to claim 15 for the treatment of Parkinson disease.

19. The molecule according to claims 1 to 12 for the treatment of Cripto-dependent tumor formation.

20. A pharmaceutical composition comprising the molecule according to claims 1 to 12 or the neuronal induced stem cells according to claim 15 and proper excipients, adjuvants and/or diluents, also associated with other therapeutic compounds wherein said other therapeutic compounds may or may not act on Cripto.

21. A pharmaceutical composition comprising the molecule according to claims 1 to 12 or the neuronal induced stem cells according to claim 15 and proper excipients, adjuvants and/or diluents to ameliorate side effects induced by other therapeutic compounds acting on Cripto.


SEQUENCE LISTING

<110>  CONSIGLIO NAZIONALE DELLE RICERCHE

<120>  CRIPTO BLOCKING MOLECULES AND THERAPEUTIC USES THEREOF

<130>  BE106267

<160>  14

<170>  PatentIn version 3.5

<210>  1
<211>  24
<212>  DNA
<213>  Artificial Sequence

<220>
<223>  synthetic primer

<400>  1
cagacctgaa ggagacctat tccc                                              24


<210>  2
<211>  24
<212>  DNA
<213>  Artificial Sequence

<220>
<223>  synthetic primer

<400>  2
gtcagcgtaa acagttgctc tacc                                              24


<210>  3
<211>  25
<212>  DNA
<213>  Artificial Sequence

<220>
<223>  synthetic primer

<400>  3
ccacgttaga ttcctcccag tgcgt                                             25


<210>  4
<211>  25
<212>  DNA
<213>  Artificial Sequence

<220>
<223>  synthetic primer

<400>  4
catacttgtt agagagttcc gtctc                                             25


<210>  5
<211>  21
<212>  DNA
<213>  Artificial Sequence

<220>
<223>  synthetic primer

<400>  5

gaaatggaag aaaccctcac a                                                21

<210>  6
<211>  23
<212>  DNA
<213>  Artificial Sequence

<220>
<223>  synthetic primer

<400>  6
ccggccttgg cctctggttt tgg                                              23

<210>  7
<211>  21
<212>  DNA
<213>  Artificial Sequence

<220>
<223>  synthetic primer

<400>  7
tgtcacgtcc ccaaggttca t                                                21

<210>  8
<211>  21
<212>  DNA
<213>  Artificial Sequence

<220>
<223>  synthetic primer

<400>  8
gggcaggccg ggtctctaag t                                                21

<210>  9
<211>  22
<212>  DNA
<213>  Artificial Sequence

<220>
<223>  synthetic primer

<400>  9
gtgggcttca ctgtcatcct ca                                               22

<210>  10
<211>  21
<212>  DNA
<213>  Artificial Sequence

<220>
<223>  synthetic primer

<400>  10
cccaggtcat caatgccacg a                                                21

<210>  11
<211>  20
<212>  DNA
<213>  Artificial Sequence

<220>

<223>  synthetic primer

<400>  11
acctgttgac ggattccaag                                                   20


<210>  12
<211>  20
<212>  DNA
<213>  Artificial Sequence

<220>
<223>  synthetic primer

<400>  12
tcatgaggaa gcgtaggtcc                                                   20


<210>  13
<211>  20
<212>  DNA
<213>  Artificial Sequence

<220>
<223>  synthetic primer

<400>  13
gggaggtgat aagcgatgaa                                                   20


<210>  14
<211>  20
<212>  DNA
<213>  Artificial Sequence

<220>
<223>  synthetic primer

<400>  14
cccaggttct agatccacca                                                   20